# EUROPEAN PATENT SPECIFICATION

(11) **EP 4 353 714 B1**
(45) Date of publication and mention of the grant of the patent: **06.05.2026**
(21) Application number: 22819871.9
(22) Date of filing: 16.03.2022
(51) Int. Cl.: C07D 317/38, G01N 30/12, G01N 30/88, G01N 30/16, G01N 30/18

(54) **GAS CHROMATOGRAPHY ANALYSIS METHODS BASED ON ETHYLENE CARBONATE SPLIT INJECTION**
GASCHROMATOGRAPHIEANALYSEVERFAHREN BASIERT AUF SPLITINJEKTION VON ETHYLENCARBONAT
PROCÉDÉS D'ANALYSE PAR CHROMATOGRAPHIE EN PHASE GAZEUSE AVEC INJECTION EN SPLIT DE CARBONATE D'ÉTHYLÈNE

(30) Priority: 09.06.2021 JP 2021096700
(43) Date of publication of application: 17.04.2024
(73) Proprietor: Asahi Kasei Kabushiki Kaisha, Tokyo 1000006 (JP)
(72) Inventor: YOKOYAMA, Masako, Tokyo 100-0006 (JP)
(74) Representative: Strehl & Partner mbB
(86) International application number: PCT/JP2022/011980
(87) International publication number: WO 2022/259681

(56) References cited:
- EP-A1- 2 374 803
- JP-A- H11 501 725
- GAGNE J P ET AL: "Development of a novel high volume band compression injector for the analysis of complex samples like toxaphene pesticide", JOURNAL OF CHROMATOGRAPHY A, ELSEVIER, AMSTERDAM, NL, vol. 1216, no. 3, 16 January 2009 (2009-01-16), pages 442 - 448, XP025840018, ISSN: 0021-9673, [retrieved on 20081001], DOI: 10.1016/J.CHROMA.2008.09.083
- KENTA YAMASHITA, TOMONORI IHARA, YOSHITOMO MINAMIDATE, RYOSUKE SASAKI, TAKAYA MATSUSHITA, MASASHI SHIBATA: "A study of methods for analyzing crystal violet lactone and carbonate esters (vinylene carbonate, fluoroethylene carbonate, ethyl methyl carbonate, propylene carbonate and diethyl carbonate) ", CCL REPORT., vol. 59, 1 January 2020 (2020-01-01), pages 113 - 118, XP009541837, ISSN: 0286-1933
- ANONYMOUS: "GC Supplies and Consumables: Splitless Liners", AGILENT - PRODUCT - GAS CHROMATOGRAPH- GC PARTS AND CONSUMABLES - GC INLET LINERS AND INLET SYSTEMS - SPLITLESS LINER, AGILENT TECHNOLOGIES, INC., JP, 20 December 2019 (2019-12-20), JP, XP009541839, Retrieved from the Internet <URL:https://www.chem-agilent.com/contents.php?id=1005953>
- ADAMS, SCOTT: "How to Choose a GC Inlet Liner: Simplify Selection Based on Injection Type", GENERAL APPLICATIONS, RESTEK, US, no. Lit. Cat.# GNAR1991-UNV, 1 January 2014 (2014-01-01), US, pages 1 - 4, XP009541840, Retrieved from the Internet <URL:https://www.bgb-info.com/files/master/Restek/SKY%20Liner/How%20to%20choose%20a%20GC%20Inlet%20Liner.pdf>

## Description

### Technical Field

The present invention relates to a gas chromatography analytical method.

### Background Art

In recent years, interest in lithium-ion secondary batteries (LiB) has been growing increasingly because they contribute to reduction of global environmental burden, such as energy saving or resource saving. The range of use of recent LiB is continually expanding up to not only batteries for mobile devices, such as smartphones and tablets, but also in-vehicle batteries for hybrid electric vehicles (HEV), electric vehicles (EV), etc., and further, batteries for energy storage. LiB is a secondary battery in which lithium ions move between a positive electrode and a negative electrode to carry out electrical charge and discharge, and it is composed of a positive electrode, a negative electrode, a separator, and an electrolyte. Highly polar ethylene carbonate is a typical electrolyte solvent because it has features (advantages) that it dissolves a lithium salt at a high concentration to cause dissociation, and in addition, it forms a coating film of nanometer order on the negative electrode surface of a carbon material to stabilize the carbon material of the negative electrode. However, there are concerns that impurities of ethylene carbonate have an influence on the battery performance during the battery charge and discharge cycle, and therefore, for the electrolyte applications, high-purity ethylene carbonate is used.

Regarding the lithium-ion battery manufacturing field, a variety of methods for measuring a hetero-alcohol content in ethylene carbonate particularly in a lithium-ion battery electrolyte has been proposed (e.g., Patent Literature 1).

EP 2 374 803 A1 discloses an analytical method comprising injecting ethylene carbonate to be analyzed into a gas chromatography analyzer.

### Citation List

### Patent Literature

Patent Literature 1: CN105319303A

### Summary of Invention

### Technical Problem

In the analysis of a purity of high-purity ethylene carbonate, it has become apparent that by decomposition of ethylene carbonate in gas chromatography analyzer, impurities, such as ethylene oxide (EO), ethylene glycol (EG), and diethylene glycol (DEG), are formed in amounts that are non-negligible as accuracy of the measurement results. On that account, an analytical method for ethylene carbonate with higher accuracy has been desired.

That is to say, it is an object of the present invention to provide a gas chromatography analytical method in which a purity of high-purity ethylene carbonate can be accurately analyzed by gas chromatography analysis.

### Solution to Problem

In order to solve the above-mentioned problem, the present inventor has earnestly studied, and as a result, he has found that a purity of high-purity ethylene carbonate can be accurately analyzed by introducing a sample into a capillary column using a single taper liner that is not packed with wool in a gas chromatography analytical method based on a split technique, and he has arrived at the present invention.

Furthermore, the present inventor has earnestly studied in order to solve the above-mentioned problem, and as a result, he has found that a purity of high-purity ethylene carbonate can be accurately analyzed by injecting molten ethylene carbonate into a gas chromatography analyzer in a gas chromatography analytical method based on a split technique, and he has arrived at the present invention.

The present invention is defined in the appended claims.

### Advantageous Effect of Invention

According to the present invention, a gas chromatography analytical method in which a purity of high-purity ethylene carbonate can be accurately analyzed by gas chromatography analysis can be provided.

### Brief Description of Drawings

[Figure 1] Figure 1 is a schematic block diagram of gas chromatography analyzer 1.
[Figure 2] Figure 2 is a schematic block diagram showing one example of a single taper liner.

### Description of Embodiment

Hereinafter, an embodiment of the present invention (also referred to as the "present embodiment" hereinafter) will be described in detail. The present invention is not limited to the present embodiment below, and can be carried out by variously modifying it within the range of the gist thereof.

The gas chromatography analytical method according to the present embodiment is a gas chromatography analytical method based on an ethylene carbonate split injection technique, including:
injecting ethylene carbonate to be analyzed into a gas chromatography analyzer,
vaporizing the injected ethylene carbonate in a single taper liner not packed with wool,
introducing a part of the vaporized ethylene carbonate into a capillary column with an inert gas flowing and allowing the ethylene carbonate to pass through the capillary column, and
detecting the ethylene carbonate having passed through the capillary column with a detector.

According to the present embodiment, the above-mentioned constitution is included, and therefore, a purity of high-purity ethylene carbonate can be accurately analyzed by gas chromatography analysis.

In the gas chromatography analysis, it is common to apply a split technique in which when separation is carried out by a capillary column, the whole amount of an injection sample after vaporization is not introduced into the capillary column, but only an amount of the sample appropriate to the retention volume of the capillary column is introduced, and the remainder is discharged from a vent together with an inert gas. In the split technique, use of a liner packed with wool is usual practice. The reason is that there are advantages that the sample is efficiently vaporized and can be analyzed with good reproducibility. Then, in the gas chromatography analysis of high-purity ethylene carbonate for electrolyte applications, the present inventor also used a wool-packed liner for split technique analysis first, but accuracy in measurement of purity and impurity concentration was not obtained. Moreover, the present inventor tried another liner for split technique analysis and a wool-packed liner for splitless technique analysis, but reproducibility in purity and impurity concentration was not obtained. It has become the established theory not to use a splitless liner not packed with wool in the split technique analysis because reproducibility is deteriorated, but the present inventor has thought that thermal decomposition or hydrolysis of ethylene carbonate is to take place on a surface of wool that is used for the purpose of efficiently carrying out vaporization. Then, the present inventor has studied a liner not packed with wool, and he has found a single taper liner for splitless analysis as a liner with a shape having good reproducibility of an analytical value. From a commonsense viewpoint, selecting a liner for a splitless technique in spite of the split technique analysis heads in the direction of deteriorating reproducibility of analysis, and is surprising. One embodiment of the present invention has a feature of use of a single taper liner not packed with wool in the gas chromatography analysis of high-purity ethylene carbonate.

The gas chromatography analytical method according to the present embodiment is a gas chromatography analytical method based on an ethylene carbonate split injection technique, including:
injecting molten ethylene carbonate to be analyzed into a gas chromatography analyzer,
vaporizing the injected ethylene carbonate in a liner,
introducing a part of the vaporized ethylene carbonate into a capillary column with an inert gas flowing and allowing the ethylene carbonate to pass through the capillary column, and
detecting the ethylene carbonate having passed through the capillary column with a detector.

According to the present embodiment, the above-mentioned constitution is included, and therefore, a purity of high-purity ethylene carbonate can be accurately analyzed by gas chromatography analysis.

The gas chromatography analysis according to the present embodiment includes introducing a part of the vaporized ethylene carbonate into a capillary column with an inert gas flowing and allowing the ethylene carbonate to pass through the capillary column. In other words, the gas chromatography analysis according to the present embodiment is an analysis based on a split injection technique. The split injection technique becomes reproducible analysis by vaporizing the analysis sample in a liner with the sample component concentration ratio unchanged and introducing a part of it into a capillary column. The components having different boiling points in a liquid of the sample can be vaporized at the same time in a liner, and the sample can be introduced with the sample component concentration ratio unchanged.

However, ethylene carbonate that is solid at room temperature does not go this way. After injection of ethylene carbonate into a gas chromatograph, there are phase transitions such as melting and vaporization, and the present inventor has suspected that on this occasion, by the endothermic phenomenon due to enthalpy of fusion, vaporization is inhibited, and thermal decomposition or hydrolysis is more liable to take place than vaporization. Then, the present inventor introduced molten ethylene carbonate into a gas chromatography analyzer, and as a result, components having different boiling points were also able to be vaporized at the same time in a liner, thermal decomposition or hydrolysis was able to be prevented, and accuracy of the gas chromatography analytical method was able to be enhanced.

In the above-mentioned embodiment, it is preferable to simultaneously carry out injection of molten ethylene carbonate into a gas chromatography analyzer and vaporization of ethylene carbonate in a single taper liner for injection that is not packed with wool. By simultaneously carrying out these and by introducing molten ethylene carbonate, vaporization in a liner is facilitated, and in addition, there is no adhesion to wool in the liner, and vaporization in the liner is more smoothly performed. According to this analytical method, therefore, synergistic effect due to the above-mentioned feature is exerted, and even better accuracy of a gas chromatography analytical method is obtained.

Hereinafter, gas chromatography analyzer 1 used in the present embodiment will be described.

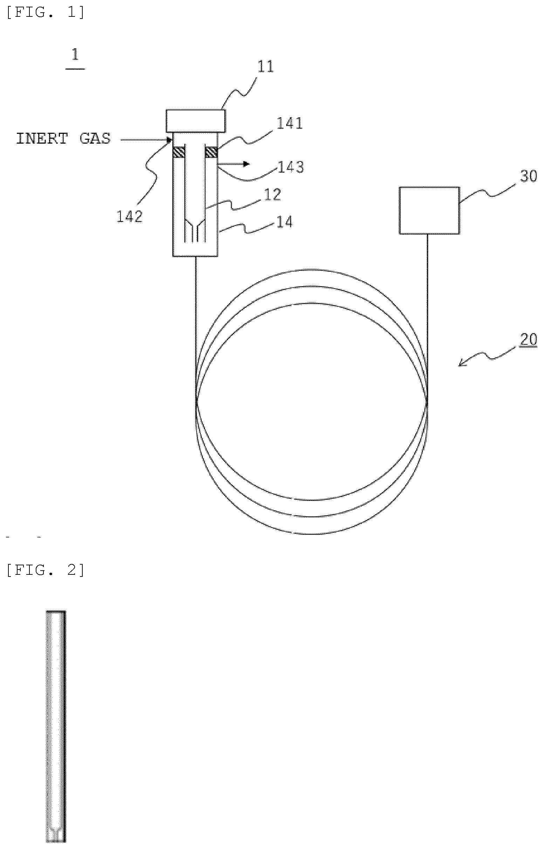

Figure 1 is a schematic block diagram of gas chromatography analyzer 1. The gas chromatography analyzer 1 includes an injection port 11, a liner 12, an injection housing 14, a capillary column 20, and a detector 30.

The injection port 11 includes a septum that is not shown in the figure, and the port is fabricated so that the sample can be injected with a microsyringe through the septum.

The liner 12 is preferably a single taper liner for injection that is not packed with wool. By using the liner, a purity of high-purity ethylene carbonate can be accurately analyzed. As the liner 12, specifically, a single taper liner shown in Figure 2 is preferably used. A material of the liner may be any of quartz and glass as long as it has been subjected to inactivation treatment. A material other than quartz and glass can be used if the high-purity ethylene carbonate for electrolyte applications and impurities contained therein are not activated and do not undergo reaction in the liner by subjecting the material to the inactivation treatment.

The wool refers to wool that is used for gas chromatography analysis, such as glass wool.

The single taper means that it has configuration in which one taper structure is provided.

The injection housing 14 fixes the liner 12 inside it through an O-ring 141. In the injection housing 14, an inert gas inlet 142 is provided. An inert gas introduced from the inert gas inlet 142 passes through the interior of the liner 12, a part of the gas is introduced into a capillary column, and the remainder is discharged from an inert gas outlet 143 provided in the injection housing 14.

The capillary column 20 is preferably a capillary column having a polar liquid phase. From the viewpoint of increasing separability of impurities contained in the ethylene carbonate, the liquid phase in the capillary column 20 preferably contains at least one component selected from the group consisting of cyanopropyl- and phenyl-substituted polysiloxane, cyanopropyl- and methyl-substituted polysiloxane, trifluoropropyl-substituted polysiloxane, phenyl-substituted polysiloxane, and polyethylene glycol. Among these, cyanopropyl- and phenyl-substituted polysiloxane, and a combination of it and dimethylsiloxane are preferable.

Regarding a column length, a column pore size, and a thickness of a liquid phase, if satisfactory separation and quantification of peaks of an actual chromatogram can be confirmed, the column length, the column pore size, and the thickness of a liquid phase can be selected from the lineup for sale.

The detector 30 is not particularly limited as long as it is a detector used for gas chromatography analysis, and examples thereof include a flame ionization detector (FID), a mass spectrometer (MS), and a micro thermal conductivity detector (µTCD).

Next, the gas chromatography analytical method according to the present embodiment will be described with reference to an example using the gas chromatography analyzer 1.

Ethylene carbonate is injected into the gas chromatography analyzer 1. The ethylene carbonate is injected from an injection port 11. Next, the injected ethylene carbonate is introduced into a liner 12 and is vaporized in the liner 12. A part of the vaporized ethylene carbonate is introduced into a capillary column 20 with an inert gas flowing and allowed to pass through the capillary column. After the ethylene carbonate is allowed to pass through the capillary column 20, it is detected with a detector 30.

The ethylene carbonate to be injected into the gas chromatography analyzer 1 is preferably in a molten state. By injecting molten ethylene carbonate, components having different boiling points can also be vaporized at the same time in the liner, thermal decomposition or hydrolysis can be prevented, and the accuracy of the gas chromatography analytical method can be enhanced.

The temperature of the molten ethylene carbonate is preferably 40°C to 80°C, more preferably 40°C to 70°C, still more preferably 40°C to 60°C.

In order to adjust the temperature of the molten ethylene carbonate, heating equipment may be used, and the temperature of the heating equipment is preferably 50°C to 90°C, more preferably 50°C to 80°C, still more preferably 50°C to 70°C. The heating equipment may be a heater that can be directly attached or can be wound, but taking into consideration operability of an autoinjector of a gas chromatograph and handling workability of a syringe, equipment that can blow warm air is preferable.

A purity of the ethylene carbonate used in the analytical method according to the present embodiment is preferably 99.5% by mass or more, more preferably 99.90% by mass or more, still more preferably 99.95% by mass or more. According to the analytical method of the present embodiment, accurate analytical results can be obtained even in the case of high-purity ethylene carbonate of 99.95% by mass or more. In the case of high-purity ethylene carbonate, determination of an accurate purity has been conventionally difficult because of thermal decomposition or hydrolysis during analysis, but the analytical method of the present embodiment enables highly accurate analysis. The purity of the ethylene carbonate is a value calculated based on the area ratio, from the measurement results obtained by the analytical method according to the present embodiment.

A water content of the ethylene carbonate used in the analytical method according to the present embodiment is preferably 100 ppm or less, more preferably 60 ppm or less, still more preferably 50 ppm or less. By using ethylene carbonate having a water content in this range, hydrolysis of the ethylene carbonate can be suppressed after the ethylene carbonate is introduced into the gas chromatography equipment, and higher accurate analysis becomes feasible. The water content can be measured by Karl Fischer method.

By carrying out an operation for suppressing moisture absorption of a sample before injection into the gas chromatography analyzer 1, a more reliable and stable analytical value can be obtained.

Sampling of the ethylene carbonate to be analyzed is carried out by, for example, collecting a small amount of ethylene carbonate from a purification tower or the like in an ethylene carbonate manufacturing apparatus.

Sampling of the ethylene carbonate is preferably carried out at 40°C or higher in order to make ethylene carbonate molten, but because of a short time, the sampling environment may be any of a dry inert gas, such as dry nitrogen or dry argon, dry air, an inert gas, air, and vacuum. Among these, a dry inert gas, dry air, or vacuum is preferable, and dry nitrogen is more preferable. Here, "dry" in the dry inert gas and the dry air means that the dew point is -20°C or lower.

The analysis sample obtained by sampling is melted in a warm air circulation oven. The temperature inside the warm air circulation oven is preferably 50 to 80°C, more preferably 50 to 70°C, still more preferably 55 to 65°C. When the temperature is in this range, ethylene carbonate can be efficiently melted while being cautious about thermal decomposition. For confirming the molten state, whether the analysis sample is melted or not should be confirmed by shaking the container with the cap closed. The environment in the warm air circulation oven may be any of air, an inert gas, dry air, and a dry inert gas if the cap is not opened. If the cap of the container is often opened when whether the analysis sample has been melted or not is confirmed, the molten ethylene carbonate easily absorbs moisture and suffers decomposition or hydrolysis. The cap should not be opened carelessly when the analysis sample obtained by sampling is in a molten state.

When an autoinjector function of the gas chromatograph is used, it is necessary to collect the molten ethylene carbonate sample with a dropping pipette and to inject it into a vial. These operations may be carried out in an environment of any of air, an inert gas, dry air, a dry inert gas, and vacuum as long as they are carried out in short time. Among these, a dry inert gas, dry air, or vacuum is preferable, and dry nitrogen is more preferable.

When the ethylene carbonate is injected into the gas chromatograph by hand using a syringe, the syringe is placed in the warm air circulation oven in advance to warm the syringe, then the cap of the container containing the sample is opened to collect the sample under, for example, a dry inert gas (preferably dry nitrogen), and the ethylene carbonate in the syringe is melted.

Before the ethylene carbonate is injected into the gas chromatography analyzer 1, an organic solvent is preferably injected into the gas chromatography analyzer 1 to clean the interior of the column. Examples of the organic solvent used herein include tetrahydrofuran. The cleaning may be carried out by introducing a part of an organic solvent having been vaporized, into a capillary column with an inert gas flowing and allowing the organic solvent to pass through the capillary column under the same conditions as the conditions for analyzing the ethylene carbonate by the gas chromatography analyzer 1. This cleaning may be repeated, for example, 3 times or more.

The installation environment of the gas chromatograph may be an air-conditioned indoor environment (e.g., 15 to 35°C). At this environmental temperature, ethylene carbonate becomes solid, so that ethylene carbonate is melted in advance as previously described, and in addition, the sample before injection into the gas chromatograph is surely melted, and it must be confirmed that the sample has been actually molten.

The injection port temperature in the gas chromatography analyzer 1 is preferably 240 to 300°C, more preferably 250 to 290°C, still more preferably 260 to 280°C.

The detector temperature in the gas chromatography analyzer 1 is 240 to 300°C, more preferably 250 to 290°C, still more preferably 260 to 280°C.

The inert gas allowed to flow in the capillary column (also referred to as a "carrier gas" hereinafter) may be nitrogen or may be helium, but it is preferably nitrogen.

The gas chromatography analysis according to the present embodiment includes introducing a part of the vaporized ethylene carbonate into a capillary column with an inert gas flowing and allowing the ethylene carbonate to pass through the capillary column, and a ratio between a flow rate of an inert gas discharged out of the capillary column and a flow rate of an inert gas introduced into the capillary column (also referred to as a "split ratio" hereinafter) is adjusted. The split ratio (flow rate of inert gas discharged out of capillary column vs flow rate of inert gas introduced into capillary column) is preferably 5:1 to 50:1, more preferably 10:1 to 40:1, still more preferably 15:1 to 30:1.

In the gas chromatography analytical method, it is preferable that a carrier gas pressure during injection of the ethylene carbonate to be analyzed into the gas chromatography analyzer be set to 1.5 to 30 times the common operating pressure, and after injection, the carrier gas pressure be set to common operating pressure. The common operating pressure is a pressure of a carrier gas at a steady flow rate of a carrier gas under the measurement conditions, and under the temperature conditions during injection. By setting the pressure during injection to 1.5 to 30 times this common operating pressure, vaporization in the liner is accelerated, and the measurement accuracy can be more enhanced.

### Examples

Hereinafter, the present invention will be more specifically described with examples, but the present invention is in no way limited to the following examples.

### [Water content]

In a syringe with needle having been dried in a dryer at 105°C and then stored in a desiccator, about 3 g of molten ethylene carbonate was collected, a tip of the needle was silicone-capped, the weight was accurately measured, thereafter the ethylene carbonate was injected into a Karl Fischer apparatus under the following conditions, a tip of the needle was silicone-capped again, and then the weight was measured. The coulomb quantity indicated by the Karl Fischer apparatus was divided by the ethylene carbonate quantity determined from the weight accurately measured, thereby determining a water content.

### <Measurement conditions>

Karl Fischer apparatus: Mitsubishi Chemical Corporation, CA-310
Anolyte: Aquamicron AX
Catholyte: Aquamicron CXU
Amount of sample injected: about 3 g

The conditions of the gas chromatography analysis carried out in Examples and Comparative Examples below are shown.
Apparatus: Agilent GC-7820A
Injection port temperature: 270°C
Injection volume: 0.4 µL
Column: DB-1701 (length 30 m × pore size (inner size) 0.53 mm × thickness 0.5 µm)
Column temperature: maintained at 100°C for 0 min, 10°C/min (100 to 120°C), 20°C/min (120 to 220°C), maintained at 220°C for 6 min
Carrier gas: nitrogen 1.0 ml/min
Common operating pressure under the temperature conditions during injection: 70195524 Pa (1.0181 psi)
Injection technique: split technique (split ratio 20:1)
Injection port liner: as described in Examples and Comparative Examples
Detector: flame ionization detector (FID) 270°C
Concentration calculation: area normalization (due to all the detected components of gas chromatograph)

### [Example 1]

Under dry air (dew point: -20°C or lower), 2 polypropylene bottles containing high-purity ethylene carbonate for electrolyte applications, which had been sampled in a molten state, were placed in a warm air circulation oven at 60°C with the caps on. It was confirmed that the whole ethylene carbonate had been melted. A water content of a sample in one polypropylene bottle was measured. Under dry nitrogen (purity: 99.99%, dew point: -80°C or lower), a sample in the other polypropylene bottle, in an amount required for the analysis, was introduced in a molten state into a vial for an autoinjector of a gas chromatograph, and the vial was capped. The vial was set in the autoinjector of the gas chromatograph, and warm air was sent so that the ethylene carbonate in the vial would not be solidified, and the ambient temperature of the vial would become about 60°C. In the gas chromatograph, a single taper liner (Agilent 5190-2292) not packed with wool was set, and gas chromatography analysis was carried out.

### [Comparative Example 1]

Under dry nitrogen (purity: 99.99%, dew point: -80°C or lower), 2 polypropylene bottles containing high-purity ethylene carbonate for electrolyte applications, which had been sampled in a molten state, were placed in a warm air circulation type at 60°C with the caps on. It was confirmed that the whole ethylene carbonate had been melted. A water content of a sample in one polypropylene bottle was measured. Under dry nitrogen (purity: 99.99%, dew point: -40°C or lower), a sample in the other polypropylene bottle, in an amount required for the analysis, was introduced in a molten state into a vial for an autoinjector of a gas chromatograph, and the vial was capped. The vial was set in the autoinjector of the gas chromatograph, and warm air was sent so that the ethylene carbonate in the vial would not be solidified, and the ambient temperature of the vial would become about 60°C. In the gas chromatograph, a focus liner (Agilent 5190-2295) packed with wool was set, and gas chromatography analysis was carried out.

### [Example 2]

Under dry air (dew point: -20°C or lower), 2 polypropylene bottles containing high-purity ethylene carbonate for electrolyte applications, which had been sampled in a molten state, were placed in a warm air circulation oven at 60°C with the caps on. It was confirmed that the whole ethylene carbonate had been melted. A water content of a sample in one polypropylene bottle was measured. Under air of a gas chromatograph installation environment, a sample in the other polypropylene bottle, in an amount required for the analysis, was introduced in a molten state into a vial for an autoinjector of a gas chromatograph, and the vial was capped. The vial was set in the autoinjector of the gas chromatograph, and warm air was sent so that the ethylene carbonate in the vial would not be solidified, and the ambient temperature of the vial would become about 60°C. In the gas chromatograph, a single taper liner (Agilent 5190-2292) not packed with wool was set, and gas chromatography analysis was carried out.

### [Example 3]

Under air, 2 polypropylene bottles containing high-purity ethylene carbonate for electrolyte applications, which had been sampled in a molten state, were placed in a warm air circulation oven at 60°C with the caps on. It was confirmed that the whole ethylene carbonate had been melted. A water content of a sample in one polypropylene bottle was measured. Under dry nitrogen (purity: 99.99%, dew point: -80°C or lower), a sample in the other polypropylene bottle, in an amount required for the analysis, was introduced in a molten state into a vial for an autoinjector of a gas chromatograph, and the vial was capped. The vial was set in the autoinjector of the gas chromatograph, and warm air was sent so that the ethylene carbonate in the vial would not be solidified, and the ambient temperature of the vial would become about 60°C. In the gas chromatograph, a single taper liner (Agilent 5190-2292) not packed with wool was set, and gas chromatography analysis was carried out.

### [Example 4]

Under dry nitrogen (purity: 99.99%, dew point: -40°C or lower), 2 polypropylene bottles containing high-purity ethylene carbonate for electrolyte applications, which had been sampled in a molten state, were placed in a warm air circulation oven at 60°C with the caps on. It was confirmed that the whole ethylene carbonate had been melted. A water content of a sample in one polypropylene bottle was measured. Under dry nitrogen (purity: 99.99%, dew point: -80°C or lower), a sample in the other polypropylene bottle, in an amount required for the analysis, was introduced in a molten state into a vial for an autoinjector of a gas chromatograph, and the vial was capped. The vial was set in the autoinjector of the gas chromatograph, and warm air was sent so that the ethylene carbonate in the vial would not be solidified, and the ambient temperature of the vial would become about 60°C. In the gas chromatograph, a single taper liner (Agilent 5190-2292) not packed with wool was set, and gas chromatography analysis was carried out.

### [Example 5]

Under dry nitrogen (purity: 99.99%, dew point: -80°C or lower), 2 polypropylene bottles containing high-purity ethylene carbonate for electrolyte applications, which had been sampled in a molten state, were placed in a warm air circulation oven at 60°C with the caps on. It was confirmed that the whole ethylene carbonate had been melted. A water content of a sample in one polypropylene bottle was measured. Under dry nitrogen (purity: 99.99%, dew point: -80°C or lower), a sample in the other polypropylene bottle, in an amount required for the analysis, was introduced in a molten state into a vial for an autoinjector of a gas chromatograph, and the vial was capped. The vial was set in the autoinjector of the gas chromatograph, and warm air was sent so that the ethylene carbonate in the vial would not be solidified, and the ambient temperature of the vial would become about 60°C. In the gas chromatograph, a single taper liner (Agilent 5190-2292) not packed with wool was set, and using pressure injection function (pressure injection conditions: pressure before starting analysis 13789.5 Pa (2 psi)) of the gas chromatograph, gas chromatography analysis was carried out.

**[Table 1]**

| | Sampling | Oven atmosphere | Transfer into vial | H20 | Pressure | EO | EG | DEG | EC | TEG |
|---|---|---|---|---|---|---|---|---|---|---|
| | | | | ppm | | ppm | ppm | ppm | % | ppm |
| Example 1 | dry air | dry air | dry nitrogen | 35 | common operating | 37 | 76 | 54 | 99.97 | 43 |
| Comparative Example 1 | dry nitrogen | dry air | dry nitrogen | 28 | common operating | 332 | 64 | 0 | 99.96 | 0 |
| Example 2 | dry air | dry air | air | 40 | common operating | 18 | 64 | 49 | 99.98 | 22 |
| Example 3 | air | dry air | dry nitrogen | 37 | common operating | 26 | 75 | 63 | 99.97 | 45 |
| Example 4 | dry nitrogen | dry air | dry nitrogen | 27 | common operating | 0 | 14 | 20 | 100.00 | 7 |
| Example 5 | dry nitrogen | dry air | dry nitrogen | 25 | pressure application during injection | 0 | 14 | 21 | 100.00 | 9 |

Meanings of abbreviations in Table 1 are as follows.
EO: ethylene oxide, EG: ethylene glycol, DEG: diethylene glycol, EC: ethylene carbonate, TEG: triethylene glycol

### Industrial Applicability

As usage of LiB expands, demand for high-purity ethylene carbonate for electrolyte applications is expected to grow more and more in the future. Accurate evaluation of a purity of high-purity ethylene carbonate and impurities therein leads to the ability to provide a high-quality electrolyte solvent, and can contribute to enhancement of battery performance.

This application claims a priority to Japanese Patent Application No. 2021-096700.

### Reference Signs List

1...gas chromatography analyzer
11...injection port
12...liner
14...injection housing
20...capillary column
30...detector
141...0-ring
142...inert gas inlet
143...inert gas outlet

## Claims

1. A gas chromatography analytical method based on an ethylene carbonate split injection technique, comprising:
injecting ethylene carbonate to be analyzed into a gas chromatography analyzer,
vaporizing the injected ethylene carbonate in a single taper liner not packed with wool,
introducing a part of the vaporized ethylene carbonate into a capillary column with an inert gas flowing and allowing the ethylene carbonate to pass through the capillary column, and
detecting the ethylene carbonate having passed through the capillary column with a detector.

2. A gas chromatography analytical method based on an ethylene carbonate split injection technique, comprising:
injecting molten ethylene carbonate to be analyzed into a gas chromatography analyzer,
vaporizing the injected ethylene carbonate in a liner,
introducing a part of the vaporized ethylene carbonate into a capillary column with an inert gas flowing and allowing the ethylene carbonate to pass through the capillary column, and
detecting the ethylene carbonate having passed through the capillary column with a detector.

3. The gas chromatography analytical method according to claim 2, wherein the liner is a single taper liner not packed with wool.

4. The gas chromatography analytical method according to any one of claims 1 to 3, wherein a water content of the ethylene carbonate to be analyzed is 100 ppm or less.

5. The gas chromatography analytical method according to any one of claims 1 to 4, wherein a purity of the ethylene carbonate to be analyzed is 99.5% by mass or more.

6. The gas chromatography analytical method according to any one of claims 1 to 5, wherein a carrier gas pressure during injection of the ethylene carbonate to be analyzed into the gas chromatography analyzer is set to 1.5 to 30 times a common operating pressure, and after injection, the carrier gas pressure is set to the common operating pressure.

7. The gas chromatography analytical method according to any one of claims 1 to 6, further comprising collecting the ethylene carbonate to be analyzed into a syringe for injection in a gas chromatograph, under a dry inert gas.

8. The gas chromatography analytical method according to any one of claims 1 to 6, further comprising introducing the ethylene carbonate to be analyzed into a vial for an autoinjector of a gas chromatograph and capping the vial, under a dry inert gas.

9. The gas chromatography analytical method according to any one of claims 1 to 8, further comprising sampling the ethylene carbonate to be analyzed under dry air, under a dry inert gas, or under vacuum.

10. The gas chromatography analytical method according to any one of claims 1 to 9, wherein a liquid phase in the capillary column contains at least one component selected from the group consisting of cyanopropyl- and phenyl-substituted polysiloxane, cyanopropyl- and methyl-substituted polysiloxane, trifluoropropyl-substituted polysiloxane, phenyl-substituted polysiloxane, and polyethylene glycol.

11. The gas chromatography analytical method according to any one of claims 1 to 10, wherein an injection port temperature in the gas chromatography analyzer is 250 to 290°C.

12. The gas chromatography analytical method according to any one of claims 1 to 11, wherein a detector temperature in the gas chromatography analyzer is 240 to 300°C.

13. The gas chromatography analytical method according to any one of claims 1 to 12, wherein the inert gas allowed to flow in the capillary column is nitrogen or helium.

14. The gas chromatography analytical method according to any one of claims 1 to 13, wherein a split ratio (flow rate of inert gas discharged out of capillary column vs flow rate of inert gas introduced into capillary column) is 5:1 to 50:1.

## Patentansprüche

1. Gaschromatographie-Analyseverfahren, welches auf einer Ethylencarbonat-Split-Injektionstechnik basiert, umfassend:
Injizieren von zu analysierendem Ethylencarbonat in einen Gaschromatographie-Analysator,
Verdampfen des injizierten Ethylencarbonats in einem nicht mit Wolle gepackten Single Taper Liner,
Einführen eines Teils des verdampften Ethylencarbonats in eine Kapillarsäule mit einem strömenden Inertgas und Ermöglichen, dass das Ethylencarbonats die Kapillarsäule durchläuft, und
Detektieren des Ethylencarbonats, welches die Kapillarsäule durchlaufen hat, mit einem Detektor.

2. Gaschromatographie-Analyseverfahren, welches auf einer Ethylencarbonat-Split-Injektionstechnik basiert, umfassend:
Injizieren von geschmolzenem, zu analysierenden Ethylencarbonat in einen Gaschromatographie-Analysator,
Verdampfen des injizierten Ethylencarbonats in einem Liner,
Einführen eines Teils des verdampften Ethylencarbonats in eine Kapillarsäule mit einem strömenden Inertgas und Ermöglichen, dass das Ethylencarbonats die Kapillarsäule durchläuft, und
Detektieren des Ethylencarbonats, welches die Kapillarsäule durchlaufen hat, mit einem Detektor.

3. Gaschromatographie-Analyseverfahren nach Anspruch 2, wobei der Liner ein nicht mit Wolle gepackter Single Taper Liner ist.

4. Gaschromatographie-Analyseverfahren nach einem der Ansprüche 1 bis 3, wobei ein Wassergehalt des zu analysierenden Ethylencarbonats 100 ppm oder weniger beträgt.

5. Gaschromatographie-Analyseverfahren nach einem der Ansprüche 1 bis 4, wobei die Reinheit des zu analysierenden Ethylencarbonats 99,5 Gew.-% oder mehr beträgt.

6. Gaschromatographie-Analyseverfahren nach einem der Ansprüche 1 bis 5, wobei ein Trägergasdruck während der Injektion des zu analysierenden Ethylencarbonats in den Gaschromatographie-Analysator auf das 1,5- bis 30-fache eines üblichen Betriebsdrucks eingestellt wird, und nach der Injektion der Trägergasdruck auf den üblichen Betriebsdruck eingestellt wird.

7. Gaschromatographie-Analyseverfahren nach einem der Ansprüche 1 bis 6, weiterhin umfassend das Aufnehmen des zu analysierenden Ethylencarbonats in eine Spritze zur Injektion in einen Gaschromatographen unter einem trockenen Inertgas.

8. Gaschromatographie-Analyseverfahren nach einem der Ansprüche 1 bis 6, weiterhin umfassend das Einführen des zu analysierenden Ethylencarbonats in ein Vial für einen Autoinjektor eines Gaschromatographen und das Verschließen des Vials unter einem trockenen Inertgas.

9. Gaschromatographie-Analyseverfahren nach einem der Ansprüche 1 bis 8, weiterhin umfassend die Probenahme des zu analysierenden Ethylencarbonats unter trockener Luft, unter einem trockenen Inertgas oder unter Vakuum.

10. Gaschromatographie-Analyseverfahren nach einem der Ansprüche 1 bis 9, wobei eine flüssige Phase in der Kapillarsäule mindestens eine Komponente enthält, ausgewählt aus der Gruppe bestehend aus Cyanopropyl- und Phenyl-substituiertem Polysiloxan, Cyanopropyl- und Methyl-substituiertem Polysiloxan, Trifluoropropyl-substituiertem Polysiloxan, Phenyl-substituiertem Polysiloxan und Polyethylenglycol.

11. Gaschromatographie-Analyseverfahren nach einem der Ansprüche 1 bis 10, wobei eine Injektionsanschluss-Temperatur in dem Gaschromatographie-Analysator 250 bis 290°C beträgt.

12. Gaschromatographie-Analyseverfahren nach einem der Ansprüche 1 bis 11, wobei eine Detektor-Temperatur in dem Gaschromatographie-Analysator 240 bis 300°C beträgt.

13. Gaschromatographie-Analyseverfahren nach einem der Ansprüche 1 bis 12, wobei das Inertgas, welches in der Kapillarsäule einströmengelassen wird, Stickstoff oder Helium ist.

14. Gaschromatographie-Analyseverfahren nach einem der Ansprüche 1 bis 13, wobei ein Split-Verhältnis (Fließrate des aus der Kapillarsäule austretenden Inertgases zur Fließrate des in die Kapillarsäule eingeführten Inertgases) 5:1 bis 50:1 beträgt.

## Revendications

1. Procédé d'analyse par chromatographie en phase gazeuse basé sur une technique d'injection fractionnée de carbonate d'éthylène, comprenant :
injection de carbonate d'éthylène à analyser dans un analyseur par chromatographie en phase gazeuse,
vaporisation du carbonate d'éthylène injecté dans un seul tube non garni de laine et comportant une partie conique,
introduction d'une partie du carbonate d'éthylène vaporisé dans une colonne capillaire avec un flux de gaz inerte et le passage du carbonate d'éthylène à travers la colonne capillaire, et
détection du carbonate d'éthylène ayant traversé la colonne capillaire à l'aide d'un détecteur.

2. Procédé d'analyse par chromatographie en phase gazeuse basé sur une technique d'injection fractionnée de carbonate d'éthylène, comprenant :
injection de carbonate d'éthylène fondu à analyser dans un analyseur par chromatographie en phase gazeuse,
vaporisation du carbonate d'éthylène injecté dans un tube,
introduction d'une partie du carbonate d'éthylène vaporisé dans une colonne capillaire avec un flux de gaz inerte et le passage du carbonate d'éthylène à travers la colonne capillaire, et
détection du carbonate d'éthylène ayant traversé la colonne capillaire à l'aide d'un détecteur.

3. Procédé d'analyse par chromatographie en phase gazeuse selon la revendication 2, dans lequel le tube est un tube unique non garni de laine et comportant une partie conique.

4. Procédé d'analyse par chromatographie en phase gazeuse selon l'une quelconque des revendications 1 à 3, dans lequel la teneur en eau du carbonate d'éthylène à analyser est de 100 ppm ou moins.

5. Procédé d'analyse par chromatographie en phase gazeuse selon l'une quelconque des revendications 1 à 4, dans lequel la pureté du carbonate d'éthylène à analyser est de 99,5 % en masse ou plus.

6. Procédé d'analyse par chromatographie en phase gazeuse selon l'une quelconque des revendications 1 à 5, dans lequel la pression du gaz vecteur lors de l'injection du carbonate d'éthylène à analyser dans l'analyseur par chromatographie en phase gazeuse est fixée de 1,5 à 30 fois la pression de fonctionnement courante, et après l'injection, la pression du gaz vecteur est fixée à la pression de fonctionnement courante.

7. Procédé d'analyse par chromatographie en phase gazeuse selon l'une quelconque des revendications 1 à 6, comprenant en outre la collecte du carbonate d'éthylène à analyser dans une seringue pour injection dans un chromatographe en phase gazeuse, sous un gaz inerte sec.

8. Procédé d'analyse par chromatographie en phase gazeuse selon l'une quelconque des revendications 1 à 6, comprenant en outre l'introduction du carbonate d'éthylène à analyser dans un flacon pour un auto-injecteur d'un chromatographe en phase gazeuse et le bouchage du flacon, sous un gaz inerte sec.

9. Procédé d'analyse par chromatographie en phase gazeuse selon l'une quelconque des revendications 1 à 8, comprenant en outre l'échantillonnage du carbonate d'éthylène à analyser sous air sec, sous un gaz inerte sec ou sous vide.

10. Procédé d'analyse par chromatographie en phase gazeuse selon l'une quelconque des revendications 1 à 9, dans lequel une phase liquide dans la colonne capillaire contient au moins un composant choisi parmi le groupe constitué de polysiloxane substitué par du cyanopropyle et du phényle, de polysiloxane substitué par du cyanopropyle et du méthyle, de polysiloxane substitué par du trifluoropropyle, de polysiloxane substitué par du phényle et de polyéthylène glycol.

11. Procédé d'analyse par chromatographie en phase gazeuse selon l'une quelconque des revendications 1 à 10, dans lequel la température du port d'injection dans l'analyseur par chromatographie en phase gazeuse est de 250 à 290 °C.

12. Procédé d'analyse par chromatographie en phase gazeuse selon l'une quelconque des revendications 1 à 11, dans lequel la température du détecteur dans l'analyseur de chromatographie en phase gazeuse est de 240 à 300 °C.

13. Procédé d'analyse par chromatographie en phase gazeuse selon l'une quelconque des revendications 1 à 12, dans lequel le gaz inerte autorisé à circuler dans la colonne capillaire est de l'azote ou de l'hélium.

14. Procédé d'analyse par chromatographie en phase gazeuse selon l'une quelconque des revendications 1 à 13, dans laquelle un rapport de division (débit de gaz inerte évacué de la colonne capillaire par rapport au débit de gaz inerte introduit dans la colonne capillaire) est de 5:1 à 50:1.
